# EUROPÄISCHE PATENTSCHRIFT

(11) **EP 1 165 534 B1**
(45) Veröffentlichungstag und Bekanntmachung des Hinweises auf die Patenterteilung: **02.02.2005**
(21) Anmeldenummer: 99952453.1
(22) Anmeldetag: 16.09.1999
(51) Int. Cl.: C07D 295/192, A61K 31/495, C07D 207/34, C07D 307/68, C07D 207/327, C07D 213/81

(54) **BENZOYLGUANIDIN-ABKÖMMLINGE MIT VORTEILHAFTEN EIGENSCHAFTEN, VERFAHREN ZU IHRER HERSTELLUNG UND IHRE VERWENDUNG BEI DER HERSTELLUNG VON ARZNEIMITTELN**
BENZOYLGUANIDINE DERIVATIVES WITH ADVANTAGEOUS PROPERTIES, METHOD FOR PRODUCING THEM AND THEIR USE IN THE PRODUCTION OF MEDICAMENTS
NOUVEAUX DERIVES DE BENZOYLGUANIDINE AUX PROPRIETES AVANTAGEUSES, LEUR PROCEDE DE PRODUCTION ET LEUR UTILISATION DANS LA PRODUCTION DE MEDICAMENTS

(30) Priorität: 22.09.1998 DE 19843489
(43) Veröffentlichungstag der Anmeldung: 02.01.2002
(73) Patentinhaber: Boehringer Ingelheim Pharma GmbH & Co.KG, 55218 Ingelheim am Rhein (DE)
(72) Erfinder: ROOS, Otto, (DE); BLECH, Stefan-Matthias, D-88447 Warthausen (DE); BÜRGER, Erich, D-55411 Bingen am Rhein (DE); EICKMEIER, Christian, D-65187 Wiesbaden (DE)
(74) Vertreter: Kläs, Heinz-Gerd, Dr.
(86) Internationale Anmeldenummer: PCT/EP1999/006857
(87) Internationale Veröffentlichungsnummer: WO 2000/017176

(56) Entgegenhaltungen:
- DE-A- 19 601 303

## Beschreibung

Die vorliegende Erfindung betrifft neuartige Benzoylguanidin-Abkömmlinge der allgemeinen Formel I, Verfahren zu ihrer Herstellung und ihre Verwendung zur Herstellung von Arzneimitteln 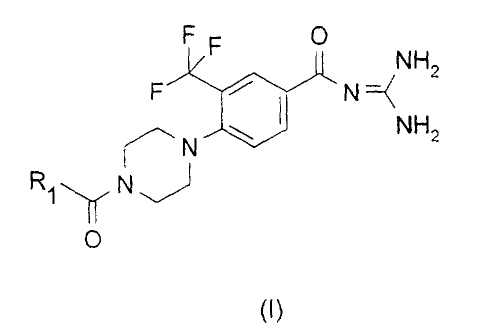 in der
- R₁: C₁-C₈-Alkyl,
Heteroaryl unsubstituiert oder ein- oder mehrfach substituiert mit einer verzweigten oder unverzweigten C₁-C₄-Alkylgrupe, einer Cycloalkylgruppe, einer verzweigten oder unverzweigten C₁-C₄-Alkoxygrupe, einer NH₂-Grupppe oder einer primären oder sekundären Aminogrupe, einer Trifluormethylgruppe einer Cyano- oder Nitrogruppe oder Halogen,
Aryl unsubstituiert oder ein- oder mehrfach substituiert mit einer verzweigten oder unverzweigten C₁-C₄-Alkylgrupe, einer verzweigten oder unverzweigten C₁-C₄-Alkoxygrupe, einer NH₂-Gruppe oder einer primären oder sekundären Aminogrupe einer Trifluormethylgruppe einer Hydroxy-, Cyano- oder Nitrogruppe oder Halogen oder mit einem 5- oder 6-gliedrigen Heteroarylrest, der ein, zwei, drei, vier oder fünf Heteroatome aus der Gruppe Stickstoff, Sauerstoff oder Schwefel - untereinander gleich oder verschieden - enthalten kann,
Alkylaryl unsubstituiert oder ein- oder mehrfach substiuiertert in der Aryl- und/oder Alkyl-Partialstruktur mit einer verzweigten oder unverzweigten C₁-C₄-Alkylgrupe, einer verzweigten oder unverzweigten C₁-C₄-Alkoxygrupe, einer NH₂-Gruppe oder einer primären oder sekundären Aminogrupe einer Trifluormethylgruppe einer Cyano- oder Nitrogruppe oder Halogen,
bedeuten kann, gegebenenfalls in Form der einzelnen Tautomeren oder gegebenenfalls Enantiomeren und deren Mischungen sowie in Form der freien Basen oder den entsprechenden Säureaddtitionssalzen mit pharmakologisch unbedenklichen Säuren.

Bevorzugt im Sinne der vorliegenden Erfindung sind Verbindungen der allgemeinen Formel I, in der
- R₁: einen unsubstituierten Phenylring oder einen Phenylring, der mit Fluor oder einer Methyl-, Trifluormethyl-, Methoxygruppe oder einem Pyrrolylrest substituiert ist, oder 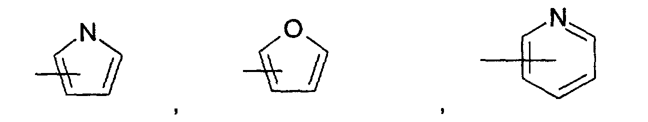
bedeuten kann.

Besonders bevorzugt sind folgende Verbindungen:
4-(4-(2-Pyrrolylcarbonyl)-1-piperazinyl)-3-trifluormethyl-benzoylguanidinmethansulfonat 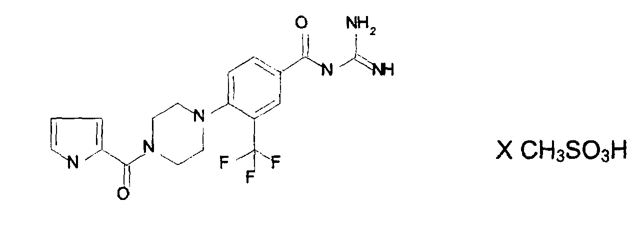 und
4-(4-(4-Fluorphenylcarbonyl)-1-piperazinyl)-3-trifluormethyl-benzoylguanidinmethansulfonat

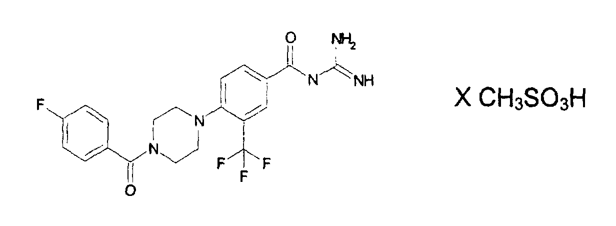

C₁-C₄-Alkyl bzw. C₁-C₈-Alkyl steht im allgemeinen für einen verzweigten oder unverzweigten Kohlenwasserstoffrest mit 1 bis 4 bzw. 8 Kohlenstoffatom(en), der gegebenenfalls mit einem oder mehreren Halogenatom(en) - vorzugsweise Fluor - substituiert sein kann, die untereinander gleich oder verschieden sein können. Als Beispiele seien folgende Kohlenwasserstoffreste genannt:
Methyl, Ethyl, Propyl, 1-Methylethyl (Isopropyl), n-Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylproypyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2,-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl. Bevorzugt sind - sofern nicht anders angegeben - Niederalkylreste mit 1 bis 4 Kohlenstoffatomen, wie Methyl; Ethyl, Propyl, *iso*-Propyl, n-Butyl, 1-Methylpropyl, 2-Methylpropyl oder 1,1-Dimethylethyl.

Alkoxy steht im allgemeinen für einen über ein Sauerstoffatom gebundenen geradkettigen oder verzweigte Alkylgruppe. Bevorzugt ist ein Niederalkoxyrest mit 1 bis 4 Kohlenstoffatom(en). - Besonders bevorzugt ist die Methoxygruppe.

Aryl steht im allgemeinen für einen aromatischen Rest mit 6 bis 10 Kohlenstoffatomen - auch in Zusammensetzungen, wobei der Aromat mit einer oder mehreren Niederalkylgruppe(n), Trifluormethylgruppe(n), Cyanogruppe(n), Alkoxygruppe(n), Nitrogruppe(n), Aminogruppe(n) und/oder einem oder mehreren Halogenatom(en) - untereinander gleich oder verschieden - substituiert sein kann; bevorzugter Arylrest ist ein gegebenenfalls substituierter Phenylrest, wobei als Substituenten Halogen - wie Fluor, Chlor oder Brom - Cyano sowie Hydroxyl bevorzugt sind - insbesondere wird im Sinne der vorliegenden Erfindung Fluor als Halogen bevorzugt. Der Arylsubstitituent - vorzugsweise Phenyl - kann des weiteren mit einem 5- oder 6-gliedrigen Heteroarylrest substituiert sein, der ein, zwei, drei, vier oder fünf Heteroatome aus der Gruppe Stickstoff, Sauerstoff oder Schwefel - untereinander gleich oder verschieden - enthalten kann.

Aralkyl steht im allgemeinen für einen über eine Alkylenkette gebundenen Arylrest mit 7 bis 14 Kohlenstoffatomen, wobei der Aromat mit einer oder mehreren Niederalkylgruppe(n), Alkoxygruppe(n), Nitrogruppe(n), Aminogruppe(n) und/oder einem oder mehreren Halogenatom(en) - untereinander gleich oder verschieden, substituiert sein kann. Bevorzugt werden Aralkylreste mit 1 bis 6 Kohlenstoffatom(en) im aliphatischen Teil und 6 Kohlenstoffatomen im aromatischen Teil.

Als bevorzugte Aralkylreste seien - sofern nicht anders angegeben - Benzyl, Phenethyl und Phenylpropyl genannt.

Halogen steht - sofern nicht anders angegeben - für Fluor, Chlor, Brom, Iod und vorzugsweise für Fluor, Chlor oder Brom.

Amino steht - sofern nicht anders angegeben - für eine NH₂-Funktion, die gegebenenfalls durch eine oder zwei C₁-C₈-Alkyl-, Aryl- oder Aralkylreste - gleich oder verschieden - substituiert sein kann.

Demgemäß steht Alkylamino zum Beispiel für Methylamino, Ethylamino, Propylamino, 1-Methylenethylamino, Butylamino, 1-Methylpropylamino, 2-Methylpropylamino oder 1,1-Dimethylethylamino.

Entsprechend steht Dialkylamino beispielsweise für Dimethylamino, Diethylamino, Dipropylamino, Dibutylamino, Di-(1-methylethyl)amino, Di-(1-methylpropyl)amino, Di-2-methylpropylamino, Ethylmethylamino, Methylpropylamino.

Cycloalkyl steht im allgemeinen für einen gesättigten oder ungesättigten cyclischen Kohlenwasserstoffrest mit 5 bis 9 Kohlenstoffatomen, der gegebenenfalls mit einem Halogenatom oder mehreren Halogenatomen - vorzugsweise Fluor - substituiert sein kann, die untereinander gleich oder verschieden sein können. Bevorzugt sind cyclische Kohlenwasserstoffe mit 3 bis 6 Kohlenstoffatomen. Als Beispiele seien Cyclopropyl, Cyclobutyl, Cyclopentyl, Cyclopentenyl, Cyclohexyl, Cyclohexenyl, Cycloheptyl, Cycloheptenyl, Cycloheptadienyl, Cyclooctyl, Cyclooctenyl, Cyclooctadienyl und Cyclononinyl genannt.

Heteroaryl im Rahmen der oben angegebenen Definition steht im allgemeinen für einen 5- bis 6-gliedrigen Ring, der als Heteroatome Sauerstoff, Schwefel und/oder Stickstoff enthalten kann und an den ein weiterer aromatischer Ring ankondensiert sein kann. Bevorzugt sind 5- und 6-gliedrige aromatische Ringe, die einen Sauerstoff, einen Schwefel und/oder bis zu zwei Stickstoffatomen enthalten und die gegebenenfalls benzokondensiert sind.

Als besondere heterocyclische Systeme seien beispielsweise Acridinyl, Acridonyl, Alkylpyridinyl, Anthrachinonyl, Ascorbyl, Azaazulenyl, Azabenzanthracenyl, Azabenzanthrenyl, Azachrysenyl, Azacyclazinyl, Azaindolyl, Azanaphthacenyl, Azanaphthalenyl, Azaprenyl, Azatriphenylenyl, Azepinyl, Azinoindolyl, Azinopyrrolyl, Benzacridinyl, Benzazapinyl, Benzofuryl, Benzonaphthyridinyl, Benzopyranonyl, Benzopyranyl, Benzopyronyl, Benzochinolinyl, Benzochinolizinyl, Benzothiepinyl, Benzothiophenyl, Benzylisoquinolinyl, Bipyridinyl, Butyrolactonyl, Caprolactamyl, Carbazolyl, Carbolinyl, Catechinyl, Chromenopyronyl, Chromonopyranyl, Cumarinyl, Cumaronyl, Decahydrochinolinyl, Decahydrochinolonyl, Diazaanthracenyl, Diazaphenanthrenyl, Dibenzazapinyl, Dibenzofuranyl, Dibenzothiphenyl, Dichromylenyl, Dihydrofuranyl, Dihydroisocumarinyl, Dihydroisochinolinyl, Dihydropyranyl, Dihydropyridinyl, Dihydropyridonyl, Dihydropyronyl, Dihydrothiopyranyl, Diprylenyl, Dioxanthylenyl, Oenantholactamyl, Flavanyl, Flavonyl, Fluoranyl, Fluoresceinyl, Furandionyl, Furanochromanyl, Furanonyl, Furanochinolinyl, Furanyl, Furopyranyl, Furopyronyl, Heteroazulenyl, Hexahydropyrazinoisoquinolinyl, Hydrofuranyl, Hydrofuranonyl, Hydroindolyl, Hydropyranyl, Hydropyridinyl, Hydropyrrolyl, Hydrochinolinyl, Hydrothiochromenyl, Hydrothiophenyl, Indolizidinyl, Indolizinyl, Indolonyl, Isatinyl, Isatogenyl, Isobenzofurandionyl, Isobenzfuranyl, Isochromanyl, Isoflavonyl, Isoindolinyl, Isoindolobenzazapinyl, Isoindolyl, Isochinolinyl, Isochinuclidinyl, Lactamyl, Lactonyl, Maleimidyl, Monoazabenzonaphthenyl, Naphthalenyl, Naphthimidazopyridindionyl, Naphthindolizinedionyl, Naphthodihydropyranyl, Naphthofuranyl, Naphthyridinyl, Oxepinyl, Oxindolyl, Oxolenyl, Perhydroazolopyridinyl, Perhydroindolyl, Phenanthrachinonyl, Phthalideisoquinolinyl, Phthalimidyl, Phthalonyl, Piperidinyl, Piperidonyl, Prolinyl, Parazinyl, Pyranoazinyl, Pyranoazolyl, Pyranopyrandionyl, Pyranopyridinyl, Pyranochinolinyl, Pyranopyrazinyl, Pyranyl, Pyrazolopyridinyl, Pyridinethionyl, Pyridinonaphthalenyl, Pyridinopyridinyl, Pyridinyl, Pyridocolinyl, Pyridoindolyl, Pyridopyridinyl, Pyridopyrimidinyl, Pyridopyrrolyl, Pyridochinolinyl, Pyronyl, Pyrrocolinyl, Pyrrolidinyl, Pyrrolizidinyl, Pyrrolizinyl, Pyrrolodioazinyl, Pyrrolonyl, Pyrrolopyrmidinyl, Pyrrolochinolonyl, Pyrrolyl, Chinacridonyl, Chinolinyl, Chinolizidinyl, Chinolizinyl, Chinolonyl, Chinuclidinyl, Rhodaminyl, Spirocumaranyl, Succinimidyl, Sulpholanyl, Sulpholenyl, Tetrahydrofuranyl, Tetrahydroisochinolinyl, Tetrahydropyranyl, Tetrahydropyridinyl, Tetrahydrothiapyranyl, Tetrahydrothiophenyl, Tetrahydrothipyranonyl, Tetrahydrothipyranyl, Tetronyl, Thiaphenyl, Thiachromanyl, Thiadecalinyl, Thianaphthenyl, Thiapyranyl, Thiapyronyl, Thiazolopyridinyl, Thienopyridinyl, Thienopyrrolyl, Thienothiophenyl, Thiepinyl, Thiochromenyl, Thiocumarinyl, Thiopyranyl, Triazaanthracenyl, Triazinoindolyl, Triazolopyridinyl, Tropanyl, Xanthenyl, Xanthonyl, Xanthydrolyl, Adeninyl, Alloxanyl, Alloxazinyl, Anthranilyl, Azabenzanthrenyl, Azabenzonaphthenyl, Azanaphthacenyl, Azaphenoxazinyl, Azapurinyl, Azinyl, Azoloazinyl, Azolyl, Barbituric Acid, Benzazinyl, Benzimidazolethionyl, Benzimidazolonyl, Benzisothiazolyl, Benzisoxazolyl, Benzocinnolinyl, Benzodiazocinyl, Benzodioxolanyl; Benzodioxolyl, Benzopyridazinyl, Benzothiazepinyl, Benzothiazinyl, Benzothiazolyl, Benzoxazinyl, Benzoxazolinonyl, Benzoxazolyl, Cinnolinyl, Depsidinyl, Diazaphenanthrenyl, Diazepinyl, Diazinyl, Dibenzoxazepinyl, Dihydrobenzimidazolyl, Dihydrobenzothiazinyl, Dihydrooxazolyl, Dihydropyridazinyl, Dihydropyrimidinyl, Dihydrothiazinyl, Dioxanyl, Dioxenyl, Dioxepinyl, Dioxinonyl, Dioxolanyl, Dioxolonyl, Dioxopiperazinyl, Dipyrimidopyrazinyl, Dithiolanyl, Dithiolenyl, Dithiolyl, Flavinyl, Furopyrimidinyl, Glycocyamidinyl, Guaninyl, Hexahydropyrazinoisoquinolinyl, Hexahydropyridazinyl, Hydantoinyl, Hydroimidazolyl, Hydroparazinyl, Hydropyrazolyl, Hydropyridazinyl, Hydropyrimidinyl, Imidazolinyl, Imidazolyl, Imidazoquinazolinyl, Imidazothiazolyl, Indazolebenzopyrazolyl, Indoxazenyl, Inosinyl, Isoalloxazinyl, Isothiazolyl, Isoxazolidinyl, Isoxazolinonyl, Isoxazolinyl, Isoxazolonyl. Isoxazolyl, Lumazinyl, Methylthyminyl, Methyluracilyl, Morpholinyl, Naphthimidazolyl, Oroticyl, Oxathianyl, Oxathiolanyl, Oxazinonyl, Oxazolidinonyl, Oxazolidinyl, Oxazolidonyl, Oxazolinonyl, Oxazolinyl, Oxazolonyl, Oxazolopyrimidinyl, Oxazolyl, Perhydrocinnolinyl, Perhydropyrroloazinyl, Perhydropyrrolothiazinyl, Perhydrothiazinonyl, Perimidinyl, Phenazinyl, Phenothiazinyl, Phenoxathiinyl, Phenoxazinyl, Phenoxazonyl, Phthalazinyl, Piperazindionyl, Piperazinodionyl, Polyquinoxalinyl, Pteridinyl, Pterinyl, Purinyl, Pyrazinyl, Pyrazolidinyl, Pyrazolidonyl, Pyrazolinonyl, Parazolinyl, Pyrazolobenzodiazepinyl, Pyrazolonyl, Pyrazolopyrimidinyl, Pyrazolotriazinyl, Pyrazolyl, Pyridazinyl, Pyridazonyl, Pyridopyrazinyl, Pyridopyrimidinyl, Pyrimidinethionyl, Pyrimidinyl, Pyrimidionyl, Pyrimidoazepinyl, Pyrimidopteridinyl, Pyrrolobenzodiazepinyl, Pyrrolodiazinyl, Pyrrolopyrimidinyl, Chinazolidinyl, Chinazolinonyl, Chinazolinyl, Chinoxalinyl, Sultamyl, Sultinyl, Sultonyl, Tetrahydrooxazolyl, Tetrahydropyrazinyl, Tetrahydropyridazinyl, Tetrahydroquinoxalinyl, Tetrahydrothiazolyl, Thiazepinyl, Thiazinyl, Thiazolidinonyl, Thiazolidinyl, Thiazolinonyl, Thiazolinyl, Thiazolobenzimidazolyl, Thiazolyl, Thienopyrimidinyl, Thiazolidinonyl, Thyminyl, Triazolopyrimidinyl, Uracilyl, Xanthinyl, Xylitolyl, Azabenzonapththenyl, Benzofuroxanyl, Benzothiadiazinyl, Benzotriazepinonyl, Benzotriazolyl, Benzoxadiazinyl, Dioxadiazinyl, Dithiadazolyl, Dithiazolyl, Furazanyl, Furoxanyl, Hydrotriazolyl, Hydroxytrizinyl, Oxadiazinyl, Oxadiazolyl, Oxathiazinonyl, Oxatriazolyl, Pentazinyl, Pentazolyl, Pentazinyl, Polyoxadiazolyl, Sydonyl, Tetraoxanyl, Tetrazepinyl, Tetrazinyl, Tetrazolyl, Thiadiazinyl, Thiadiazolinyl, Thiadiazolyl, Thiadioxazinyl, Thiatriazinyl, Thiatriazolyl, Thiatriazolyl, Triazepinyl, Triazinoindolyl, Triazinyl, Triazolinedionyl, Triazolinyl, Triazolyl, Trioxanyl, Triphenodioxazinyl, Triphenodithiazinyl, Trithiadiazepinyl, Trithianyl, oder Trioxolanyl genannt.

Verbindungen dieses Typs sind aus der Deutschen Offenlegungsschrift 196 01 303.8 bereits bekannt.

Derartige Verbindungen sind infolge ihrer Wirkung als Inhibitoren des zellulären Na⁺/H⁺-Austausches zur Herstellung von Arzneimitteln mit einer inhibitorischen Wirkung bezüglich des Na+/H+ Austauschs verwendbar oder können als Zwischenprodukte zur Herstellung solcher Wirkstoffe Verwendung finden. Die erfindungsgemäßen Verbindungen wirken gegen Arrhythmien, die beispielsweise bei Hypoxien auftreten. Sie sind ferner anwendbar bei Krankheiten, die im Zusammenhang mit Ischämien stehen (Beispiele: cardiale, cerbrale, gastrointestinale - wie mensenteriale Throbose/Embolie -, pulmonale, renale Ischämie, Ischämie der Leber, Ischämie der Skelettmuskulatur). Entsprechende Krankheiten sind beispielsweise coronare Herzerkrankung, Herzinfarkt, Angina pectoris, stabile Angina Pectoris, ventrikuläre Arrythmien, subventrikuläre Arrythmien, Herzinsuffizienz - ferner zur Unterstützung von Bypass-Operationen, zur Unterstützung von Operationen am offenen Herzen, zur Unterstützung von Operationen, die eine Unterbrechung der Blutversorgung des Herzens erforderlich machen und zur Unterstützung von Herztransplatationen - Embolie im Lungenkreislauf, akutes oder chronisches Nierenversagen, chronische Niereninsuffizienz, Hirninfarkt, Reperfusionsschäden bei der Wiederdurchblutung von Hirnarealen nach Auflösung von Gefäßverschlüssen und akute und chronische Durchblutungsstörungen des Hirns. Hier sind die genannten Verbindungen auch in Kombination mit thrombolytischen Mitteln wie t-PA, Streptokinase und Urokinase nützlich.

Bei der Reperfusion des ischämischen Herzens (z. B. nach einem Angina-pectoris-Anfall oder einem Herzinfarkt) können irreversible Schädigungen an Cardiomyocyten in der betroffenen Region auftreten. Die erfindungsgemäßen Verbindungen wirken u. a. in einem solchen Fall cardioprotektiv.

In das Anwendungsgebiet Ischämie ist auch die Verhinderung von Schäden an Transplantaten einzubeziehen (z. B. als Schutz des Transplantates - wie beispielseweise Leber, Niere, Herz oder Lunge - vor, während und nach der Implantation sowie bei der Lagerung der Transplantate), die im Zusammenhang mit Transplantationen auftreten können. Die Verbindungen sind außerdem protektiv wirkende Arzneimittel bei der Durchführung angioplastischer operativer Eingriffe am Herzen und an peripheren Gefäßen.

Bei der essentiellen Hypertonie und diabetischen Nephropathie ist der zelluläre Natrium-Protonen-Austausch erhöht. Die erfindungsgemäßen Verbindungen eignen sich daher als Inhibitoren dieses Austausches zur vorbeugenden Behandlung dieser Krankheiten.

Die erfindungsgemäßen Verbindungen zeichnen sich weiterhin durch eine stark inhibierende Wirkung auf die Proliferation von Zellen aus. Deshalb sind die Verbindungen als Arzneimitteln bei Krankheiten interessant, bei denen die Zellproliferation eine primäre oder sekundäre Rolle spielt und können als Mittel gegen Krebserkrankungen, gutartige Tumoren, oder beispielsweise Prostatahypertrophie, Atherosklesose, Organhypertrophien und -hyperplasien, fibrotische Erkrankungen und diabetische Spätkomplikationen verwendet werden.

Des weiteren ist von Verbindungen dieses Typs bekannt, daß sie einen günstigen Einfluß auf die Blutspiegel der Serumlipoproteine nehmen können.

Überraschenderweise wurde nun gefunden, daß die Verbindungen der allgemeinen Formel 1 gegenüber denjenigen aus dem Stand der Technik bereits bekannten Benzoylguanidin-Derivaten, den Vorzug haben daß sie neben einer unerwartet höheren Wirksamkeit den Vorteil einer oralen Verfügbarkeit in sich bergen.

Die Wirkstoffe gemäß der allgemeinen Formel I können als wässerige Injektionslösung (z.B. für intravenöse, intramuskuläre oder subkutane Applikation), als Tablette, als Suppositorium, als Salbe, als Pflaster für transdermale Applikation, als Aerosol für inhalative Anwendung über die Lunge oder als Nasenspray eingesetzt werden.

Der Wirkstoffgehalt einer Tablette oder eines Suppositoriums liegt zwischen 5 und 200 mg, vorzugsweise zwischen 10 und 50 mg. Bei Inhalation liegt die Einzeldosis zwischen 0,05 und 20 mg, vorzugsweise zwischen 0,2 und 5 mg. Bei einer parenteralen Injektion liegt die Einzeldosis zwischen 0,1 und 50 mg, vorzugsweise zwischen 0,5 und 20 mg. Die genannten Dosen können, falls erforderlich, mehrmals täglich gegeben werden.

Im folgenden sind einige Beispiele für pharmazeutische Präparate mit dem Wirkstoff angegeben:

| Tabletten: | |
|---|---|
| Wirkstoff gemäß der allgemeinen Formel I | 20,0 mg |
| Magnesiumstearat | 1,0 mg |
| Maisstärke | 62,0 mg |
| Laktose | 83,0 mg |
| Polyvinylpyrolidon | 1,6 mg |

| Lösung zur Injektion | |
|---|---|
| Wirkstoff gemäß der allgemeinen Formel I | 0,3 g |
| Natriumchlorid | 0,9 g |
| Aqua injectibilia | ad 100ml |

Die Lösung kann unter Verwendung von Standardverfahren sterilisiert werden.

| Wäßrige Lösung zur nasalen oder inhalativen Applikation | |
|---|---|
| Wirkstoff gemäß der allgemeinen Formel I | 0,3 g |
| Natriumchlorid | 0,9 g |
| Benzalkoniumchlorid | 0,01 mg |
| Aqua purificata | ad 100 ml |

Die oben ausgeführte Lösung ist geeignet für die nasale Applikation in einem Spray, oder in Kombination mit einem Gerät, das ein Aerosol mit einer Partikelgröße vorzugsweise zwischen 2 und 6 µm produziert, zur Anwendung über die Lunge.

### Kapseln für die Inhalation

Die Verbindungen der allgemeinen Formle 1 werden in mikronisierter Form (Partikelgröße im wesentlichen zwischen 2 und 6 µM), gegebenenfalls unter Zusatz von mikronisierten Trägersubstanzen, etwa Laktose, in Hartgelatinekapseln gefüllt. Zur Inhalation dienen übliche Geräte für die Pulverinhalation. In jede Kapsel werden z.B. zwischen 0,2 und 20 mg des Wirkstoffs der allgemeinen Formel I und 0 bis 40 mg Laktose eingefüllt.

| Inhalationsaerosol | |
|---|---|
| Wirkstoff gemäß der allgemeinen Formel I | 1 Teil |
| Sojalecithin | 0,2 Teile |
| Treibgasmischung | ad 100 Teile |

Die Zubereitung wird vorzugsweise in Aerosolbehälter gefüllt mit Dosierventil abgefüllt, der einzeine Hub wird so bemessen, daß eine Dosis von 0,5 mg abgegeben wird. Für die anderen Dosierungen des angegeben Bereichs verwendet man zweckmäßig Zubereitungen mit höherem oder niedrigerem Wirkstoffanteil.

| Salbe (Zusammensetzung g/100 g Salbe) | |
|---|---|
| Wirkstoff gemäß der allgemeinen Formel I | 2g |
| Rauchende Salzsäure | 0,011 g |
| Natriumpyrosulfit | 0,05 g |
| Gemisch aus gleichen Teilen Cetylalkohol und Stearylalkohol | 20 g |
| Weiße Vaseline | 5 g |
| Künstliches Bergamotteöl | 0,075 g |
| Destilliertes Wasser | ad 100 |

Die Bestandteile werden in üblicher Weise zu einer Salbe verarbeitet.

Die Herstellverfahren für die erfindungsgemäßen Verbindungen sind aus dem Stand der Technik allgemein bekannt; so können die erfindungsgemäßen Verbindungen beispielsweise auf folgendem Wege gewonnen werden:

Durch Umsetzung 4-(1-Piperazinyl)-3-trifluomethylbenzoesäureestern der allgemeinen Formel II 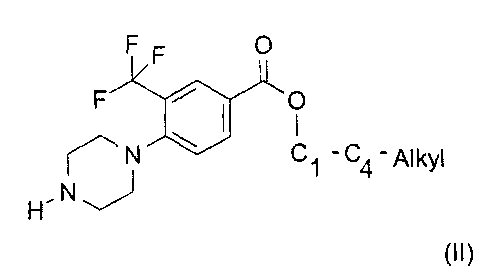 mit einer Verbindung der allgemeinen Formel III,

R₁C(O)Q (III)

in der Q eine durch den Piperazin-Stickstoff substituierbare Abgangsgruppe bedeutet, ggf. in Anwesenheit von Hilfsstoffen, vorzugweise Carbonyldiimidazol, erhält man das resultierende Benzoesäurederivat der allgemeinen Formel IV 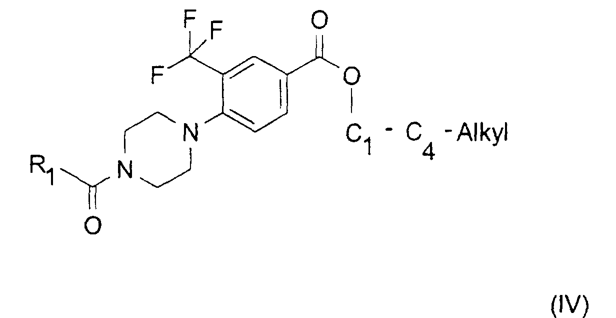 das man in einem geeigneten - vorzugsweise wasserfreien - Lösungsmittel - bevorzugt Dimethylformamid - suspendiert und mit der Mischung einer Lösung oder Suspension einer Base - vorzugsweise Natriumhydrid in einem geeigenten, wasserfreiem Lösungsmittel - vorzugsweise Dimetylformamid - mit einem Guanidinsalz - vozugsweise Guanidinhydrochlorid - versetzt und das Reaktionsprodukt isoliert.

Die vorliegende Erfindung wird durch die nachfolgenden Beispiele erläutert:

### Beispiele:

### 4-Fluor-3-trifluormethyl-benzoesäuremethylester

35.4 g (170 mmol) 4-Fluor-3-(trifluormethyl)-benzoesäure in 250 ml Methanol werden unter Eiskühlung bei 5°C innerhalb von 25 Minuten mit 68 ml SOCl₂ versetzt. Nach vollendeter Zugabe wird das Reaktionsgemisch noch über einen Zeitraum von 3 h am Rückfluß erhitzt. Die Reaktionslösung wird auf Raumtemperatur abgekühlt und i.Vak. eingedampft. Der ölige Rückstand wird in 200 ml Diethylether aufgenommen und mit Wasser, gesättigter NaHCO₃-Lösung und wieder mit Wasser extrahiert. Die vereinigten organischen Phasen werden über Magnesiumsulfat getrocknnet und i.Vak. eingedampft.

Ausbeute: 29.0 g (77%)

### 4-(4-Benzyl-1-piperazinyl)-3-trifluormethyl-benzoesäuremethylester

7 g (31.5 mmol) 4-Fluor-3-trifluormethyl-benzoesäuremethylester werden in 60 ml trockenem Dimethylsulfoxid (DMSO) gelöst und mit 5.55 g (31.5 mmol) N-Benzylpiperazin und 4.35 g (31.5 mmol) Kaliumcarbonat versetzt. Die Mischung wird 12 h bei 90°C gerührt. Nach Abkühlen wird die Reaktionsmischung in 200 ml Wasser gegossen und dreimal mit Essigsäurethylester (Essigester) extrahiert. Die vereinigten organischen Phasen werden mit Wasser und gesättigter Natriumchlorid-Lösung gewaschen, über Magnesiumsulfat getrocknet und i.Vak. abdestilliert. Der Rückstand wird mit einem Essigester/n-Heptan-Gemisch an Kieselgel chromatographiert.

Ausbeute: 3.93 g (33%)

### 4-(1-Piperazinyl)-3-trifluormethyl-benzoesäuremethylester

20.2 g (53.3 mmol) 4-(4-Benzyl-1-piperazinyl)-3-trifluormethylbenzoesäuremethylester werden in 200 ml Methanol gelöst und mit 2 g Palladium auf Kohle versetzt und über einen Zeitraum von 1.4 h bei 70°C und unter einem Wasserstoffdruck von 5 bar hydriert. Die Lösung wird über Cellit abgesaugt und i.Vak. abdestilliert.

Ausbeute: 14.85 g (97%)

### Allgemeine Vorschrift zur Kupplung von 4-(1-Piperazinyl)-3-trifluormethylbenzoesäuremethylester mit Benzoesäuren:

5 mmol der entsprechenden Carbonsäure werden in 30 ml absolutem Tetrahydrofuran (THF) gelöst und unter Schutzgas bei 0°C mit 810 mg (5 mmol) Carbonyldiimidazol versetzt und 2 h bei Raumtemperatur (ca. 25 °C) gerührt. Anschließend gibt man 1.44 g (5 mmol) 4-(1-Piperazinyl)-3-trifluormethylbenzoesäuremethylester zu und rührt über einen Zeitraum von ca. 12 h weiter. Die Lösung wird i.Vak. bis zur Trockene eingedampft und mit Essigester aufgenommen. Nach dem Waschen mit gesättigter NaHCO₃-Lösung, gesättigter NaCl-Lösung und Wasser werden die organischen Phasen über MgSO₄ getrocknet und i.Vak. eingedampft. Nach Kristallisation in einem geeigneten Lösungsmittel oder Chromatographie an Kieselgel mit einem geeigneten Laufmittel erhält man folgende Verbindungen.
1. 4-(4-(3-Methoxyphenylcarbonyl)-1-piperazinyl)-3-trifiuormethylbenzoesäuremethylester
   Säulenchromatographie: Essigester/n-Heptan (2:1)
   Ausbeute: 81%
2. 4-(4-(2-Pyrrolylcarbonyl)-1-piperazinyl)-3-trifluormethyl-benzoesäuremethylester
   Kristallisation aus Methanol
   Ausbeute: 75%
   Smp.: 149°C
3. 4-(4-(4-Fluorphenylcarbonyl)-1-piperazinyl)-3-trifluormethylbenzoesäuremethylester
   Säulenchromatographie: Essigester/n-Heptan (2:1)
   Ausbeute: 77%
4. 4-(4-(2-Methoxyphenylcarbonyl)-1-piperazinyl)-3-trifluormethylbenzoesäuremethylester
   Säulenchromatographie: Essigester/n-Heptan (2:1)
   Ausbeute: 79%
5. 4-(4-(3-Trifluormethylphenylcarbonyl)-1-piperazinyl)-3-trifluormethylbenzoesäuremethylester
   Säulenchromatographie: Essigester/n-Heptan (2:1)
   Ausbeute: 83%
6. 4-(4-Phenylcarbonyl-1-piperazinyl)-3-trifluormethyl-benzoesäuremethylester
   Säulenchromatographie: Essigester/n-Heptan (2:1)
   Ausbeute: 87%
7. 4-(4-(2-Furylcarbonyl)-1-piperazinyl)-3-trifluormethyl-benzoesäuremethylester
   Säulenchromatographie: Essigester/n-Heptan (2:1)
   Ausbeute: 75%
8. 4-(4-(3-Methylphenylcarbonyl)-1-piperazinyl)-3-trifluormethylbenzoesäuremethylester
   Säulenchromatographie: Essigester/n-Heptan (2:1)
   Ausbeute: 79%
9. 4-(4-(4-(1-Pyrryl)phenylcarbonyl)-1-piperazinyl)-3-trifluormethylbenzoesäuremethylester
   Säulenchromatographie: Essigester/n-Heptan (2:1)
   Ausbeute: 87%
10. 4-(4-(2-Pyridylcarbonyl)-1-piperazinyl)-3-trifluormethyl-benzoesäuremethylester
   Säulenchromatographie: Essigester/n-Heptan (2:1)
   Ausbeute: 73%

### Allgemeine Vorschrift zur Herstellung der Acylguanidine aus den entsprechenden Carbonsäuremethylestern:

5.09 g (127.2 mmol) 60 %iges NaH in Weißöl wird zweimal mit Ether gewaschen und abdekantiert. 200 ml absolutes DMF werden zugegeben und unter Rühren und Schutzgas werden 12.15 g (127.2 mmol) Guanidinhydrochlorid in kleinen Portionen zugegeben. Nach einstündigem Rühren werden 21.2 mmol des entsprechenden Methylesters zugegeben und die Lösung noch über einen Zeitraum von 2 h bei einer Temperatur von ca. 120 °C gerührt. Anschließend läßt man das Reaktiongemisch auf Raumtemperatur abkühlen, filtriert und dampft das Filtrat i.Vak. ein. Nach der Chromatographie an Kieselgel mit einem geeigneten Laufmittel und Überführung mit etherischer Salzsäure oder anderen pharmakologisch verträglichen Säuren in die entsprechenden Salze erhält man die nachfolgenden Verbindungen (in den nachhfolgenden Strukturformeln sind die Wasserstoffatome aus Gründen der Übersichtlichkeit weggelssen worden, sofern sie an ein Kohlenstoff oder Stickstoffatom gebunden sind und sofern sie für das Verständnis der Erfindung nicht erforderlich sind):
**1. Beispiel** 4-(4-(3-Methoxyphenylcarbonyl)-1-piperazinyl)-3-trifluormethylbenzoylguanidin-hydrochlorid 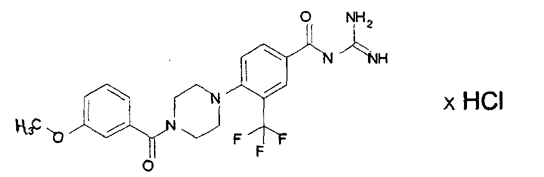 aus 4-(4-(3-Methoxyphenylcarbonyl)-1-piperazinyl)-3-trifluormethylbenzoesäuremethylester
   Säulenchromatographie: Essigester/Methanol (5:1)
   Ausbeute: 71 %
   Smp.: >200°C
   MS: (M+H)⁺ = 450 (freie Base)
**2. Beispiel** 4-(4-(2-Pyrrolylcarbonyl)-1-piperazinyl)-3-trifluormethylbenzoylguanidin-methansulfonat 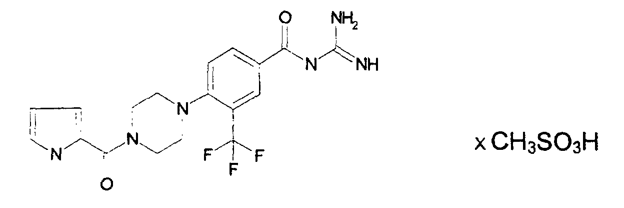 aus 4-(4-(2-Pyrrolylcarbonyl)-1-piperazinyl)-3-trifluormethyl-benzoesäuremethylester Säulenchromatographie: Essigester/Methanol (5:1)
   Ausbeute: 66%
   Smp.: 246°C
   MS: (M+H)⁺ = 409 (freie Base)
**3. Beispiel** 4-(4-(4-Fluorphenylcarbonyl)-1-piperazinyl)-3-trifluormethylbenzoylguanidin-methansulfonat 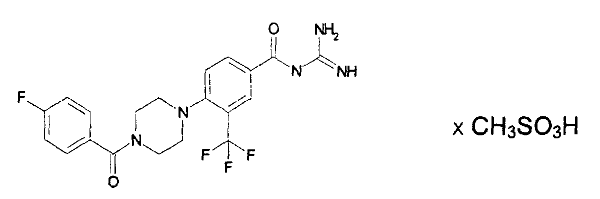 aus 4-(4-(4-Fluorphenylcarbonyl)-1-piperazinyl)-3-trifluormethylbenzoesäuremethylester
   Säulenchromatographie: Essigester:Methanol (5:1)
   Ausbeute:40%
   Smp.: 140°C
   MS: (M+H)⁺ = 438 (freie Base)
**4. Beispiel** 4-(4-(2-Methoxyphenylcarbonyl)-1-piperazinyl)-3-trifluormethylbenzoylguanidin-hydrochlorid 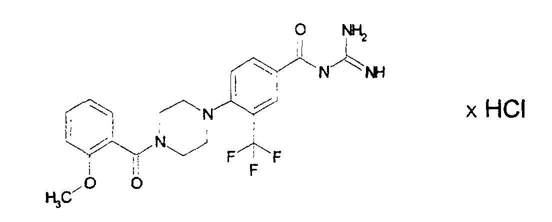 aus 4-(4-(2-Methoxyphenylcarbonyl)-1-piperazinyl)-3-trifluormethylbenzoesäuremethylester
   Säulenchromatographie: Essigester:Methanol (5:1)
   Ausbeute:71 %
   Smp.: 219°C (Zersetzung)
   MS: (M+H)⁺ = 450 (freie Base)
**5. Beispiel** 4-(4-(3-Trifluormethylphenylcarbonyl)-1-piperazinyl)-3-trifluormethylbenzoylguanidin-hydrochlorid 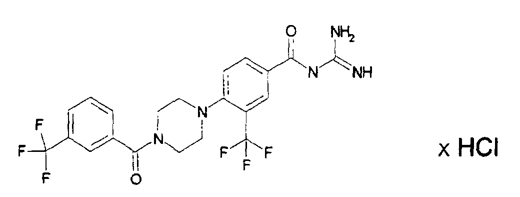 aus 4-(4-(3-Trifluormethylphenylcarbonyl)-1 -piperazinyl)-3-trifluormethylbenzoesäuremethylester
   Säulenchromatographie: Essigester:Methanol (5:1)
   Ausbeute:25%
   Smp.: 140°C (Zersetzung)
   MS: (M+H)⁺ = 488 (freie Base)
**6. Beispiel** 4-(4-Phenylcarbonyl-1-piperazinyl)-3-trifluormethyl- benzoylguanidinhydrochlorid 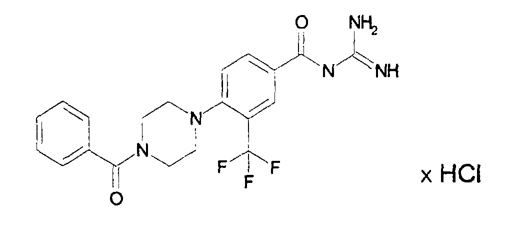 aus 4-(4-Phenylcarbonyl-1-piperazinyl)-3-trifluormethyl-benzoesäuremethylester Säulenchromatographie: Essigester:Methanol (5:1)
   Ausbeute:64%
   Smp.: 214°C
   MS: (M+H)⁺ = 420 (freie Base)
**7. Beispiel** 4-(4-(2-Furylcarbonyl)-1-piperazinyl)-3-trifluormethyl- benzoylguanidinmethansulfonat 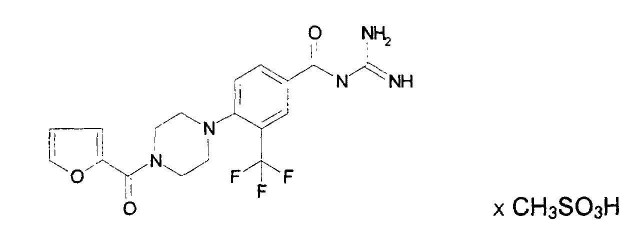 aus 4-(4-(2-Furylcarbonyl)-1-piperazinyl)-3-trifluormethyl-benzoesäuremethylester Kristallisation aus Ether
   Ausbeute:19%
   Smp.: 190°C (Zersetzung)
   MS: (M+H)⁺ = 410 (freie Base)
**8. Beispiel** 4-(4-(3-Methylphenylcarbonyl)-1-piperazinyl)-3-trifluormethylbenzoylguanidin-methansulfonat 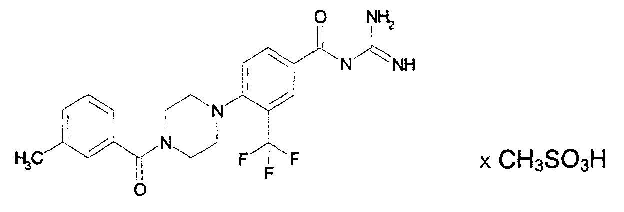 aus 4-(4-(3-Methylphenylcarbonyl)-1-piperazinyl)-3-trifluormethylbenzoesäuremethylester
   Kristallisation aus Methanol/Essigester
   Ausbeute:76%
   Smp.: 199°C
   MS: (M+H)⁺ = 434 (freie Base)
**9. Beispiel** 4-(4-(4-(1-Pyrrolyl)phenylcarbonyl)-1-piperazinyl)-3-trifluormethylbenzoylguanidin-dimethansulfonat 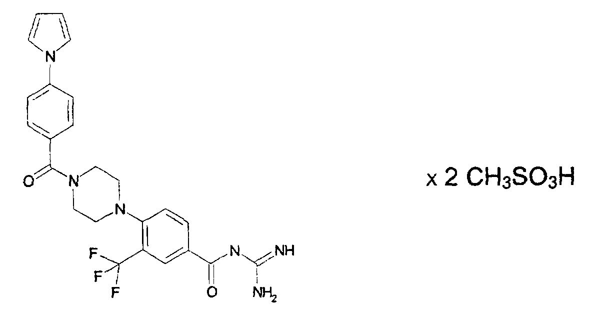 aus 4-(4-(4-(1-Pyrryl)phenylcarbonyl)-1-piperazinyl)-3-trifluormethylbenzoesäuremethylester
   Kristallisation aus Methanol
   Ausbeute:48%
   Smp.: 150°C (Zersetzung)
   MS: (M+H)⁺ = 485 (freie Base)
**10. Beispiel** 4-(4-(2-Pyridylcarbonyl)-1-piperazinyl)-3-trifluormethyl- benzoylguanidindimethansulfonat 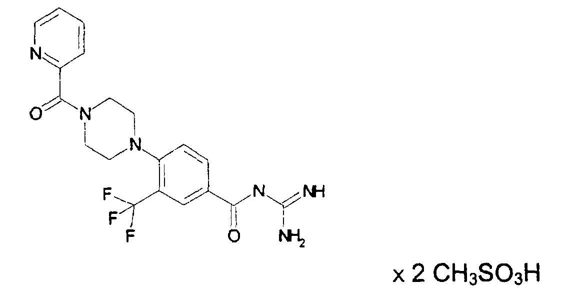 aus 4-(4-(2-Pyridylcarbonyl)-1-piperazinyl)-3-trifluormethyl-benzoesäuremethylester Säulenchromatographie: Essigester:Methanol (5:1)
   Ausbeute:34%
   Smp.: 115°C (Zersetzung)
   MS: (M+H)⁺ = 421 (freie Base)

### Pharmakologische Daten:

### Inhibition des Na⁺/H⁺-Austauschers in humanen Darmkrebszellen (HT-29):

HT-29-Zellen werden bei 37°C, 5% CO₂ in Wachstumsmedium inkubiert. Nach 3-5 Tagen wurde das Wachstumsmedium entfernt, die Zellen gewaschen und mit 7.5 µM BCECF-AM (pH-sensitiver Fluoreszenzfarbstoff) bei 37°C ohne CO₂ beladen. Nach 30 min werden die Zellen gewaschen und mit folgendem Medium angesäuert: 70 mM Cholinchlorid, 20 mM NH₄Cl, 1 mM MgCl₂, 1.8 mM CaCl₂, 5 mM Glucose und 15 mM HEPES, pH 7.5.

Nach 6 min Inkubation bei 37°C ohne CO₂ werden die Zellen gewaschen und für 5 min mit Waschmedium inkubiert: 120 mM Cholinchlorid, 5 mM KCl, 1 mM MgCl₂, 1.8 mM CaCl₂, 5 mM Glucose und 15 mM MOPS, pH 7.0.

Das Waschmedium wird entfernt und Kontrollmedium mit oder ohne Testverbindung wird zugegeben: 120 mM NaCl, 5 mM KCl, 1 mM MgCl₂, 1.8 mM CaCl₂, 5 mM Glucose, 15 mM MOPS, pH 7.0.

Die Zellen werden 4 min bei 37°C ohne CO₂ inkubiert und fluorimetrisch (CytoFluor 2350) vermessen. Die Fluoreszenz des Farbstoffes BCECF wird bei den Anregungswellenlängen 485 nm (pH-sensitiv) und 440 nm (nicht pH-sensitiv) und bei der Emissionswellenlänge 530 nm gemessen. Der cytoplasmatische pH wird aus dem Verhältnis der Fluoreszenzen bei 485 und 440 nm berechnet. Das Fluoreszenzverhältnis wird durch Messung des Fluoreszenzsignals nach Äquilibrierung von externem und internem pH mit Nigericin kalibriert.

| **Beispiel** | **IC**_{**50**} **/ 10**^{**-6**} **mol l**^{**-1**} |
|---|---|
| 1 | 0.076 |
| 3 | 0.038 |
| 4 | 0.084 |
| 5 | 0.023 |
| 7 | 0.084 |
| 8 | 0.061 |
| 10 | 0.079 |

Die erfindungsgemäßen Verbindungen weisen darüberhinaus überraschenderweise eine sehr gute Bioverfügbarkeit und lange Halbwertszeiten nach oraler Gabe auf-Eigenschaften, die sie für eine orale Applikation hervorragend geeignet machen.

### Pharmakokinetische Daten:

Für die Untersuchungen wurden männliche, ca. 200 g schwere Ratten (nicht nüchtern), verwendet. Zur intravenösen und oralen Applikation werden die Substanzen in einer angesäuerten wässerigen Lösung (pH 3) gelöst. Einzelne Bolus Injektionen (0.5 mg/kg i.v., 2.5 mg/kg p.o.) werden in die Schwanzvene injiziert (0.2 ml/200g) oder über eine Kanüle in den Magen gegeben (1 ml/200 g). Die Applikationslösungen werden analysiert, um die applizierte Dosis zu bestätigen. 0.5 ml Aliquots Blut werden vom retro-orbitalen Venenplexus unter kurzer Halothan-Betäubung mit heparinisierten Glass-Kapillaren nach folgendem Schema entnommen:
- nach i.v. Applikation: 5 min, 15 min, 30 min, 1 h, 2 h, 4 h, 6 h, 8h;
- nach oraler Applikation: 15 min, 1 h, 2 h, 4 h, 6 h, 8 h, 24 h, 32 h.

Die Proben werden zentrifugiert und das Plasma bis zur Analyse bei - 20°C gelagert. Die Proben-Vorbereitung erfolgt durch flüssig-flüssig Extraktion mit einem internen Standard. Die Plasmaextrakte werden mit Reversed Phase HPLC, gekoppelt mit einem Elektrospray-Tandem-Massenspektrometer analysiert.
Die pharmakokinetischen Daten werden aus den entsprechenden PlasmaKonzentrationen durch Kompartiment-freie Analyse mit dem TopFit Programm bestimmt (Heinzel, G., Woloszczak, R., Thomann, P. TopFit 2.0 - Pharmacokinetic and pharmacodynamic data analysis, system for the PC, Gustav Fischer Verlag, Stuttgart, Jena, New York, 1993).

| **Beispiel** | **F** | **t**_{**1/2**} **(i.v.)** | **t**_{**1/2**} **(p.o.)** |
|---|---|---|---|
| 2 | 63 | 1.3 | 5.0 |
| 3 | 71 | 3.1 | 5.4 |
| 5 | 58 | 5.4 | 7.5 |

## Patentansprüche

1. Benzoylguanidin-Derivate der allgemeinen Formel I, 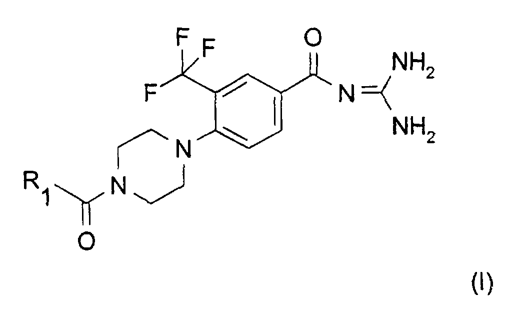 in der
R₁ C₁-C₈-Alkyl,
Heteroaryl unsubstituiert oder ein oder mehrfach substituiert mit einer verzweigten oder unverzweigten C₁-C₄-Alkylgrupe, einer Cycloalkylgruppe, einer verzweigten oder unverzweigten C₁-C₄-Alkoxygrupe, einer NH₂-Grupppe oder einer primären oder sekundären Aminogrupe, einer Trifluormethylgruppe einer Cyano- oder Nitrogruppe oder Halogen,
Aryl unsubstituiert oder ein oder mehrfach substituiert mit einer verzweigten oder unverzweigten C₁-C₄-Alkylgrupe, einer verzweigten oder unverzweigten C₁-C₄-Alkoxygrupe, einer NH₂-Grupppe oder einer primären oder sekundären Aminogrupe einer Trifluormethylgruppe einer Cyano- oder Nitrogruppe oder Halogen oder mit einem 5- oder 6-gliedrigen Heteroarylrest, der ein, zwei, drei, vier oder fünf Heteroatome aus der Gruppe Stickstoff, Sauerstoff oder Schwefel - untereinander gleich oder verschieden - enthalten kann,
Alkylaryl unsubstituiert oder ein- oder Mehrfach substiuiertert in der Aryl- und/oder Alkyl-Partialstruktur mit einer verzweigten oder unverzweigten C₁-C₄-Alkylgrupe, einer verzweigten oder unverzweigten C₁-C₄-Alkoxygrupe, einer NH₂-Grupppe oder einer primären oder sekundären Aminogrupe einer Trifluormethylgruppe einer Cyano- oder Nitrogruppe oder Halogen,
bedeuten kann gegebenenfalls in Form der einzelnen Tautomeren oder gegebenenfalls Enantiomeren und deren Mischungen sowie in Form der freien Basen oder den entsprechenden Säureaddtitionssalzen mit pharmakologisch unbedenklichen Säuren.

2. Verbindungen der allgemeinen Formel I nach Anspruch1, **dadurch gekennzeichnet, daß**
R₁ einen unsubstituierten Phenylring oder einen Phenylring, der mit Fluor oder einer Methyl-, Trifluormethyl-, einer Methoxygruppe oder mit einem Pyrrolylrest substituiert sein kann, oder 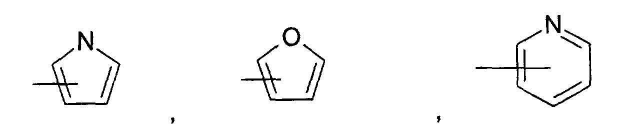
bedeuten kann.

3. 4-(4-(2-Pyrrolylcarbonyl)-1-piperazinyl)-3-trifluormethyl-benzoylguanidinmethansulfonat

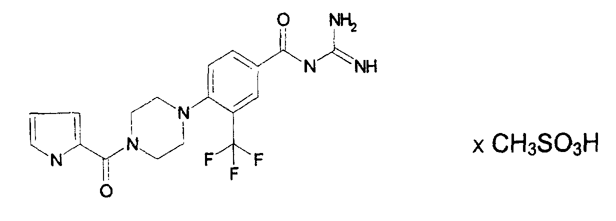

4. 4-(4-(4-Fluorphenylcarbonyl)-1-piperazinyl)-3-trifluormethyl-benzoylguanidinmethansulfonat

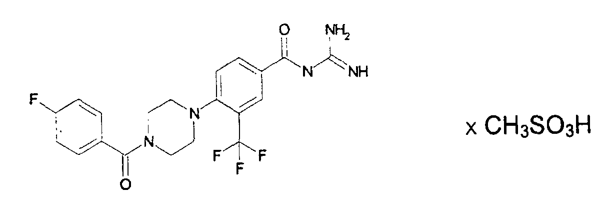

5. Verfahren zur Herstellung von Verbindungen der allgemeinen Formel I 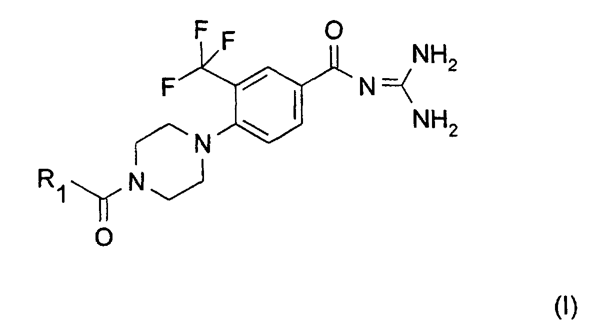 **dadurch gekennzeichnet, daß** man 4-(1-Piperazinyl)-3-trifluomethylbenzoesäureester der allgemeinen Formel II 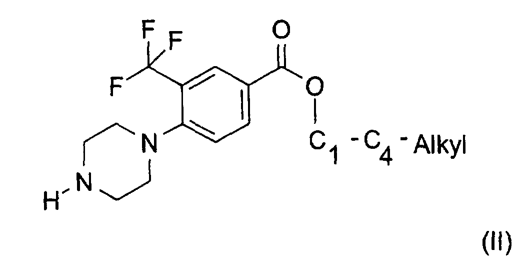 mit einer Verbindung der allgemeinen Formel III,
R₁C(O)Q (III)
in der Q eine durch den Piperazin-Stickstoff substituierbare Abgangsgruppe bedeutet, ggf. in Anwesenheit von Hilfsstoffen, vorzugweise Carbonyldiimidazol, umsetzt und das resultierende Benzoesäurederivat der allgemeinen Formel IV 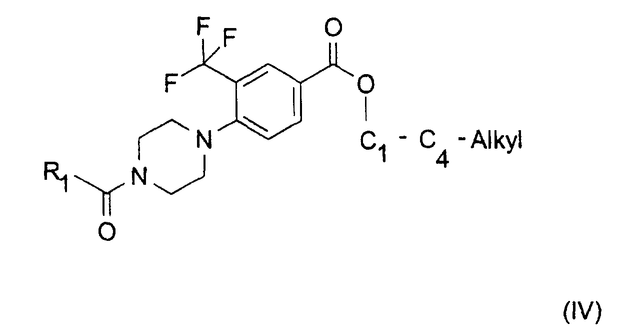 in einem geeigneten - vorzugsweise wasserfreien - Lösungsmittel - bevorzugt Dimethylformamid - suspendiert und mit der Mischung einer Lösung oder Suspension einer Base - vorzugsweise Natriumhydrid in einem geeigneten, wasserfreiem Lösungsmittel - vorzugsweise Dimetylformamid - mit einem Guanidinsalz - vorzugsweise Guanidinhydrochlorid - versetzt und das Reaktionsprodukt isoliert und gegebenenfalls mit einer pharmakologisch unbedenklichen Säure das gewünschte Säureadditionssalz bildet.

6. Pharmazeutische Zubereitung, **gekennzeichnet durch** einen Gehalt an einer Verbindung nach einem der Ansprüche 1 bis 4 und deren Säureadditionssalze neben üblichen Hilfs- und Trägersstoffen.

7. Verbindungen nach einem der Ansprüche 1 bis 4 zur Verwendung als Arzneimittel.

8. Verwendung von Verbindungen nach einem der Ansprüche 1 bis 4 zur Herstellung eines Arzneimittels mit einer inhibitorischen Wirkung bezüglich des Na+/H+ Austauschs.

9. Verwendung von Verbindungen der allgemeinen Formel I, deren Säureadditionssalze zur Herstellung eines Medikaments zur therapeutischen Behandlung von Ischaemien.

## Claims

1. Benzoylguanidine derivatives of general formula I, 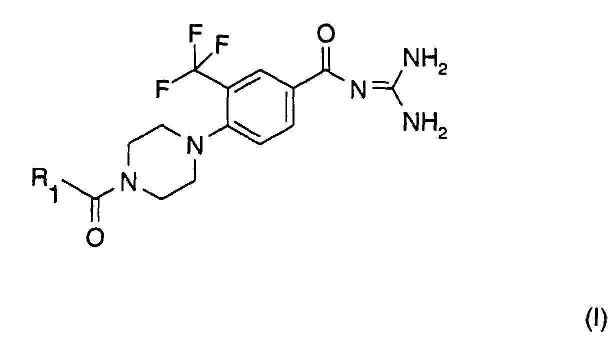 wherein
R₁ denotes C₁₋₈-alkyl,
heteroaryl, unsubstituted or mono- or polysubstituted by a branched or unbranched C₁₋₄-alkyl group, a cycloalkyl group, a branched or unbranched C₁₋₄-alkoxy group, an NH₂ group or a primary or secondary amino group, a trifluoromethyl group, a cyano or nitro group or halogen,
aryl, unsubstituted or mono- or polysubstituted by a branched or unbranched C₁₋₄-alkyl group, a branched or unbranched C₁₋₄-alkoxy group, an NH₂ group or a primary or secondary amino group, a trifluoromethyl group, cyano or nitro group or halogen or by a 5- or 6-membered heteroaryl group which may contain one, two, three, four or five heteroatoms selected from nitrogen, oxygen and sulphur - identical to one another or different
alkylaryl, unsubstituted or mono- or polysubstituted in the aryl and/or alkyl partial structure by a branched or unbranched C₁₋₄-alkyl group, a branched or unbranched C₁₋₄-alkoxy group, an NH₂ group or a primary or secondary amino group, a trifluoromethyl group, a cyano or nitro group or halogen,
optionally in the form of the individual tautomers or optionally enantiomers and mixtures thereof and in the form of the free bases or the corresponding acid addition salts with pharmacologically acceptable acids.

2. Compounds of general formula I according to claim 1, **characterised in that**
R₁ may denote an unsubstituted phenyl ring or a phenyl ring which may be substituted by fluorine or by a methyl, trifluoromethyl, methoxy group or by a pyrrolyl group, or

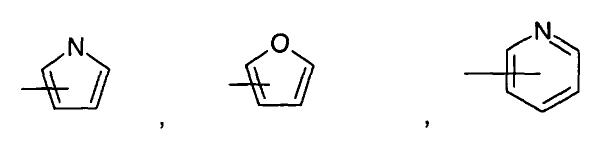

3. 4-(4-(2-Pyrrolylcarbonyl)-1-piperazinyl)-3-trifluoromethyl-benzoylguanidine-methanesulphonate

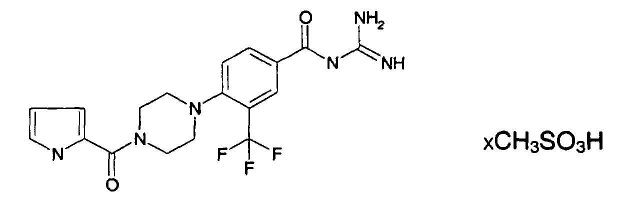

4. 4-(4-(4-Fluorophenylcarbonyl)-1-piperazinyl)-3-trifluoromethylbenzoylguanidine-methanesulphonate

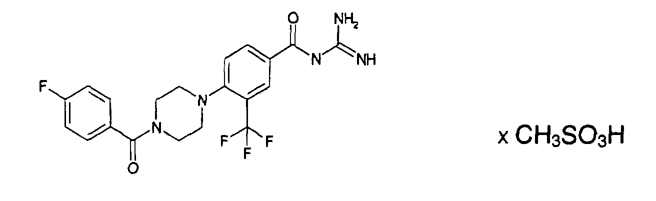

5. Process for preparing compounds of general formula I 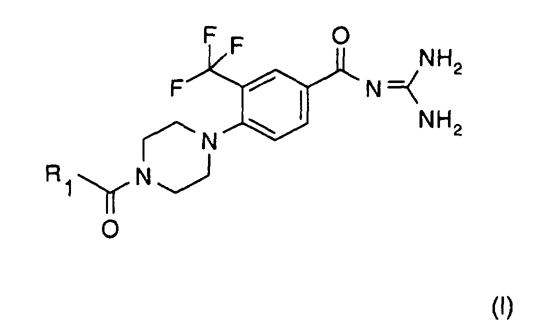 **characterised in that** a 4-(1-piperazinyl-3-trifluoromethylbenzoic acid ester of general formula II 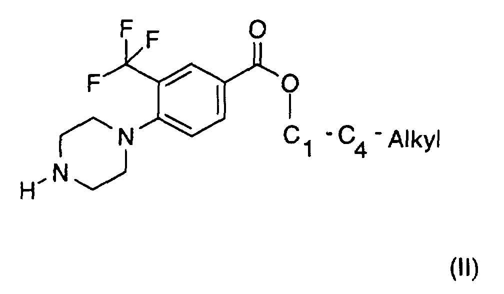 is reacted with a compound of general formula III
R₁C(O)Q (III)
in which Q denotes a leaving group which may be substituted by the piperazine nitrogen, optionally in the presence of adjuvants, preferably carbonyldiimidazole, and the resulting benzoic acid derivative of general formula IV 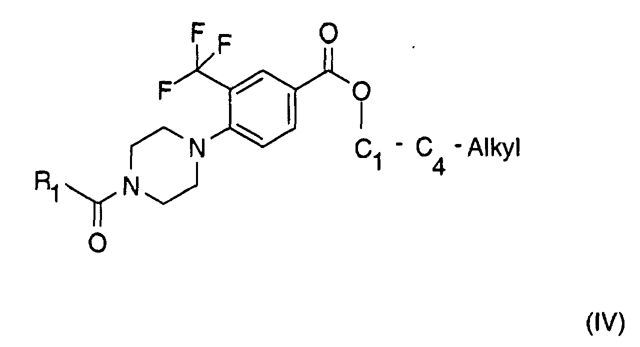 is suspended in a suitable, preferably anhydrous, solvent, preferably dimethylformamide, and is mixed with a mixture of a solution or suspension of a base - preferably sodium hydride in a suitable anhydrous solvent - preferably dimethylformamide - with a guanidine salt - preferably guanidine hydrochloride - and the reaction product is isolated and, optionally, the desired acid addition salt is formed with a pharmacologically acceptable acid.

6. Pharmaceutical preparation, **characterised in that** it contains a compound according to one of claims 1 to 4 and the acid addition salts thereof together with conventional excipients and carriers.

7. Compounds according to one of claims 1 to 4 for use as pharmaceutical compositions.

8. Use of compounds according to one of claims 1 to 4 for preparing a pharmaceutical composition having an inhibitory effect on the Na⁺/H⁺ exchange.

9. Use of compounds of general formula I and the acid addition salts thereof for preparing a medicament for the therapeutic treatment of ischaemias.

## Revendications

1. Dérivés de benzoylguanidine de formule générale I 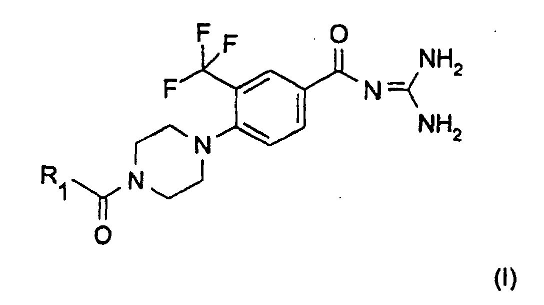 où
R₁ peut représenter C₁₋₈-alkyle,
hétéroaryle non substitué ou substitué une ou plusieurs fois avec un groupe C₁₋₄-alkyle ramifié ou non ramifié, un groupe cycloalkyle, un groupe C₁₋₄-alcoxy ramifié ou non ramifié, un groupe NH₂ ou un groupe amino primaire ou secondaire, un groupe trifluorométhyle un groupe cyano ou nitro ou halogène,
aryle non substitué ou substitué une ou plusieurs fois avec un groupe C₁₋₄-alkyle ramifié ou non ramifié, un groupe C₁₋₄-alcoxy ramifié ou non ramifié, un groupe NH₂ ou un groupe amino primaire ou secondaire un groupe trifluorométhyle un groupe cyano ou nitro ou halogène ou avec un groupement hétéroaryle à 5 ou 6 chaînons qui peut contenir un, deux, trois, quatre ou cinq hétéroatomes du groupe azote, oxygène ou soufre, identiques ou différents les uns des autres,
alkylaryle non substitué ou substitué une ou plusieurs fois dans la structure partielle aryle et/ou alkyle avec un groupe C₁₋₄-alkyle ramifié ou non ramifié, un groupe C₁₋₄-alcoxy ramifié ou non ramifié, un groupe NH₂ ou un groupe amino primaire ou secondaire un groupe trifluorométhyle un groupe cyano ou nitro ou halogène,
éventuellement sous forme des différents tautomères ou éventuellement d'énantiomères et de leurs mélanges ainsi que sous forme des bases libres ou des sels d'addition d'acide correspondants avec des acides pharmacologiquement acceptables.

2. Composés de formule générale I selon la revendication 1 **caractérisés en ce que**
R₁ peut représenter un cycle phényle non substitué ou un cycle phényle qui peut être substitué avec le fluor ou un groupe méthyle, trifluorométhyle, un groupe méthoxy ou avec un groupement pyrrolyle, ou

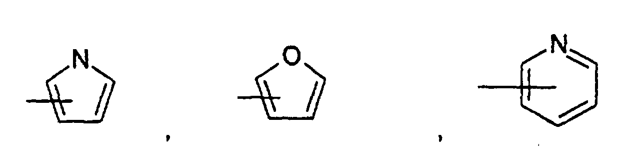

3. Méthanesulfonate de 4-(4-(2-pyrrolylcarbonyl)-1-pipérazinyl)-3-trifluorométhyl-benzoylguanidine

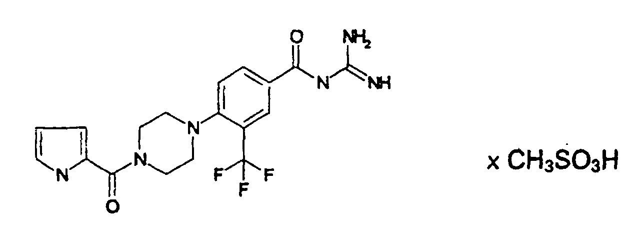

4. Méthanesulfonate de 4-(4-(4-fluorophénylcarbonyl)-1-pipérazinyl)-3-trifluorométhyl-benzoylguanidine

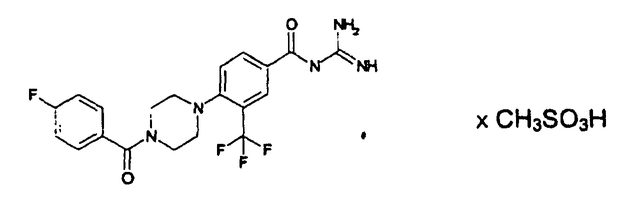

5. Procédé de préparation de composés de formule générale I 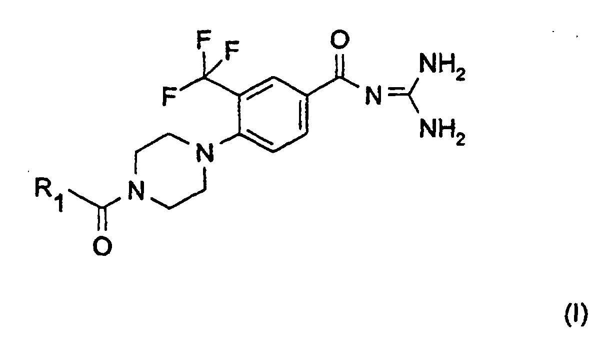 **caractérisé en ce que** l'on fait réagir un ester d'acide 4-(1-pipérazinyl)-3-trifluorométhylbenzoïque de formule générale II 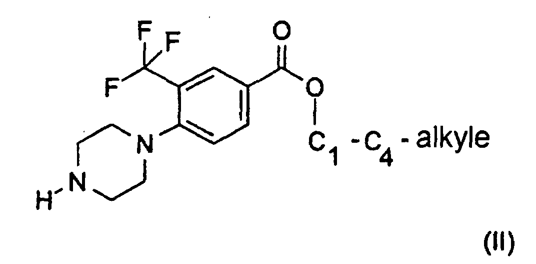 avec un composé de formule générale III
R₁C(O)Q (III)
où Q représente un groupe partant substituable par l'azote de pipérazine, éventuellement en présence d'adjuvants, de préférence de carbonyldiimidazole, et on met en suspension le dérivé d'acide benzoïque de formule générale IV 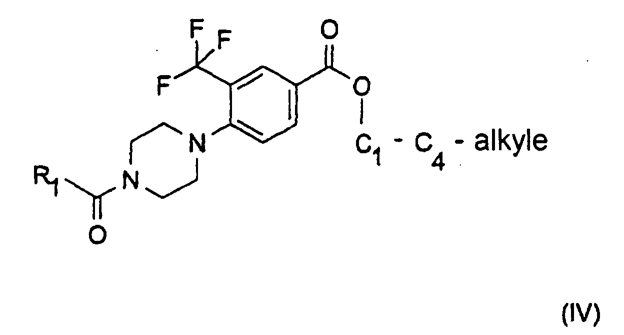 résultant dans un solvant approprié, de préférence anhydre, de préférence le diméthylformamide, et on ajoute le mélange d'une solution ou suspension d'une base, de préférence l'hydrure de sodium dans un solvant anhydre approprié, de préférence le diméthylformamide, avec un sel de guanidine, de préférence le chlorhydrate de guanidine, et on isole le produit réactionnel et on forme éventuellement le sel d'addition d'acide souhaité avec un acide pharmacologiquement acceptable.

6. Préparation pharmaceutique **caractérisée par** une teneur en un composé selon l'une des revendications 1 à 4 et ses sels d'addition d'acide outre des adjuvants et supports habituels.

7. Composés selon l'une des revendications 1 à 4 destinés à être utilisés comme médicament.

8. Utilisation de composés selon l'une des revendications 1 à 4 pour la préparation d'un médicament ayant une action inhibitrice concernant l'échange Na+/H+.

9. Utilisation de composés de formule générale I, de leurs sels d'addition d'acide pour la préparation d'un médicament pour le traitement thérapeutique d'ischémies.
